Europäisches Patentamt

(19) European Patent Office

Office européen des brevets

(11) Publication number: **0 005 286**
**A1**

## (12) EUROPEAN PATENT APPLICATION

(21) Application number: 79200171.1

(22) Date of filing: 09.04.79

(51) Int. Cl.²: **A 61 L  13/00**
// C02B3/08, A61L9/01

(30) Priority: 18.04.78  US 897478

(71) Applicant: THE PROCTER & GAMBLE COMPANY, 301 East Sixth Street, Cincinnati Ohio 45217 (US)

(43) Date of publication of application: 14.11.79
Bulletin 79/23

(72) Inventor: Nyquist, John Daniel, 23 Nickerson Road, Orleans, Massachusetts 02653 (US)

(84) Designated Contracting States: BE CH DE FR GB IT NL SE

(74) Representative: Ernst, Hubert et al, PROCTER & GAMBLE EUROPEAN TECHNICAL CENTER Temselaan 100, B-1820 Strombeek-Bever (BE)

(54) Compact solid disinfecting composition containing a mixture of lithium hypochlorite and calcium hypochlorite.

(57) A solid desinfecting composition comprising a mixture of 0.47 to 0.17 part of lithium hypochlorite for 1 part calcium hypochlorite. This composition also preferably contains inorganic diluent salts. This compositions releases chlorine desinfectant on contacting water. It is designed for use in aqueous environments, especially in toilet bowl tanks.

EP  0 005 286  A1

ACTORUM AG

COMPACT SOLID DISINFECTING COMPOSITION CONTAINING
A MIXTURE OF LITHIUM HYPOCHLORITE AND CALCIUM
HYPOCHLORITE

The present invention is directed to a solid disinfecting composition which comprises a mixture of lithium hypochlorite and calcium hypochlorite. More particularly, this invention pertains to a composite lithium hypochlorite/calcium hypochlorite solid article which supplies chlorine disinfectant to aqueous medium. The solid composition may be in the shape of a tablet, wafer, bar, disc, square, or any conventionally-shaped solid articles. It may optionally contain inorganic diluent salts which are compatible with the release of disinfectant to the aqueous environment. The lithium hypochlorite/calcium hypochlorite solid slowly dissolves on contacting water, releasing chlorine disinfectant into the aqueous environment.

Hypochlorite compositions as sources of deodorizing chlorine gases are well known in the art. U.S. Patent 3,446,893 (Hanford and Newman), issued May 27, 1969, discloses a solid hypochlorite combination with polyolefin plus a gas generating source which allows for quick release of chlorinous

gases as the solid floats on water. U.S. Patent 3,793,211 (Kohlhepp and Jass), issued February 19, 1974, discloses a lithium hypochlorite effervescent composition for use as a cleansing powder. U.S. Patent 3,276,949 (Robson and Hodges), issued October 4, 1966, discloses a compound calcium hypochlorite tablet as a chlorine generating source in wash compositions.

While lithium hypochlorite and calcium hypochlorite are desirable as available chlorine sources, each has some unfavorable features. Calcium hypochlorite compositions tend not to dissolve completely and leave a residue of insoluble inorganics. This may clog the dispensing mechanism and diminish the amount of active chlorine disinfectant which might be released to the aqueous environment. Lithium hypochlorite dissolves better in cold water, while calcium hypochlorite dissolves better in warm water. Lithium hypochlorite is a relatively scarce hypochlorite source, requiring more time and energy consuming methods to prepare.

According to the present invention, it has been found that by combining lithium hypochlorite and calcium hypochlorite within finite weight ratios in a compacted cake, a much more effective solid source for delivering active chlorine disinfectant to aqueous systems is produced than if either of these compounds is used alone. This compacted solid is extremely effective for dispensing chlorine in a controlled aqueous environment such as a toilet bowl. This composition dispenses a steady level of chlorine over a period of time and over a wide range of water temperature. This composition dissolves more evenly than prior art calcium hypochlorite compositions. The compositions of the invention also form less insoluble inorganic residue than compositions based upon calcium hypochlorite as the sole hypochlorite source. This reduction in insoluble residue reduces the tendency to cause clogging of toilet tank dispensers and also provides for more efficient delivery of all the active chlorine disinfectant in these compositions to aqueous systems. Additionally, this composition may be feasibly incorporated into any conventional dispensing mechanism for contact with an aqueous environment. In a toilet tank environment, this development allows for better control of disinfectant dispensed to the toilet water medium with each flush.

Accordingly, it is an object of the present invention to provide an improved composition for sanitizing water.

It is another object of the present invention to steadily sanitize water over a period of time.

It is still another object of the present invention to steadily sanitize water over a wide range of temperature.

It is yet another object of the present invention to provide a sanitizing composition which will dissolve substantially, leaving little residue.

It is still yet another object of the present invention to provide a more effective disinfecting composition for efficiently delivering all the available disinfectant in such composition to an aqueous environment.

It is even another object of the present invention to improve disinfecting of toilet water.

It is yet even another object of the present invention to control the level of disinfectant dispensed to the toilet water with each flush.

It is a further object of the present invention to conserve energy and natural resources by providing for a solid disinfecting composition which is parsimoniously prepared.

## Summary

The present invention is directed to a compact solid disinfecting composition containing from about 10% to about 107% by weight available chlorine, said available chlorine being supplied from a mixture of lithium hypochlorite and calcium hypochlorite wherein the weight ratio of lithium hypochlorite:calcium hypochlorite is from about 0.47:1 to about 0.17:1.

Percentages and ratios throughout the specification and claims are by weight unless otherwise stated. Temperatures are in degrees Farenheit unless otherwise indicated.

## Detailed Description of the Invention

The basic ingredient of the compositions of this invention is a solid combination of lithium hypochlorite (LiOCl) and calcium hypochlorite (Ca[OCl]$_2$). The weight ratio of lithium hypochlorite:calcium hypochlorite is from about 0.47:1 to about 0.17:1, and preferably from about 0.37:1 to about 0.29:1. The compositions of the invention contain from about 10% to about 107%, preferably from about 20% to about 45% by weight of available chlorine (AvCl$_2$). The compositions also preferably contain inorganic diluent salts.

These solid combinations containing lithium hypochlorite and calcium hypochlorite are compacted into cakes of various sizes, shapes, or forms such as tablets, wafers, bars, discs, squares, and the like. Hypochlorite is an excellent source of chlorine disinfectant, and these particular shaped articles are especially useful in dispensers designed to deliver disinfectants into toilet tanks. These compact, solid compositions may be included in toilet tank dispensers described in the following U. S. Patents, incorporated herein by reference: U.S. Patent 3,952,339 (Baur et al.), issued April 27, 1976; U.S. Patent 3,778,849 (Foley), issued December 18, 1973; and U.S. Patent 3,769,640 (Castronovo), issued November 6, 1973. The disinfecting composition, in the form of a compacted cake, is placed inside a chamber of a dispenser which is in turn placed inside the feed tank for a bathroom toilet bowl. Initially, water flows into this chamber of the dispenser, contacting the lithium hypochlorite/calcium hypochlorite cake which begins to dissolve, releasing chlorine disinfectant in the water, thereby forming a concentrated solution of hypochlorite in the dispenser chamber. When the toilet is flushed, a portion of the solution in this dispenser is discharged into the flush tank and is carried by flush water into the toilet bowl, delivering active chlorine disinfectant to the toilet bowl environment.

Lithium hypochlorite dissolves faster in colder water, while calcium hypochlorite dissolves faster in warmer water. In addition, lithium hypochlorite leaves smaller amounts of insoluble inorganics on dissolving than does calcium hypochlorite, while calcium hypochlorite is a more readily available chlorine source. By blending the two hypochlorite sources within specified weight ratios, it has been found that certain benefits may be optimized in a disinfecting composition, which are not possible when either lithium or calcium hypochlorite is used alone as a source of available chlorine. There is a greater amount of control over the level of dispensing chlorine disinfectant. There is better control over the amount of chlorine disinfectant delivered to the toilet water medium with each flush. The solid combination of lithium hypochlorite/calcium hypochlorite provides delivery of a constant amount of chlorine to the toilet water medium over a period of time. The blended solid compositions dissolve more completely than just tablets of calcium hypochlorite alone. The blended solid compositions also allow the dissolution rates of the hypochlorites, particularly the lithium hypochlorite, to be controlled over time. Blending these two chlorine sources allows for a much more efficient delivery of all available chlorine from these sources into an aqueous medium. Finally, the blending of these two hypochlorites allows for conserving lithium hypochlorite, a relatively scarce hypochlorite source.

An especially desirable advantage obtained with the compositions of this invention is the sustained, steady release of chlorine over a wide range of toilet water temperatures. The temperature of water supplied in the bathroom toilet system can vary over an extremely wide range depending on the season of the year. It has previously been extremely difficult to provide for sustained release of active chlorine disinfectant to the toilet water medium over this wide range of varying temperatures. It has now been found that by blending lithium hypochlorite and calcium hypochlorite in solid compositions, a more even rate of dissolution may be maintained over a broad temperature range (40°F-80°F), hence a more even rate of chlorine delivery to the toilet water over these ranges. This improved, steady release of chlorine over a temperature range is achieved with this solid, compacted combination over just lithium hypochlorite alone, or just calcium hypochlorite alone. It is a development which the prior art has heretofore been unable to accomplish.

## Lithium Hypochlorite

Lithium hypochlorite, $LiOCl$, can be obtained as a pure compound, but is normally commercially sold in the form of granular compositions containing about 30% to about 40% $LiOCl$ with the balance inorganic diluents and moisture. The compound $LiOCl$ contains about 120% available chlorine, thus a composition containing about 30% lithium hypochlorite contains about 36% available chlorine.

A commercial lithium hypochlorite containing about 36% available chlorine is marketed under the trade name "Form 2" by the Lithium Corporation of America. A typical chemical analysis of Form 2, as reported by the manufacturer, is as follows:

| Ingredient | Typical Weight Percent |
|---|---|
| Available Chlorine | 36 |
| Lithium Hypochlorite | 30 |
| Lithium | 4.5 |
| Sodium | 18 |
| Potassium | 3 |
| Chlorides (Total) | 45 |
| Sulfates | 11 |
| Chlorates | 2 |
| Carbonates | 1.5 |
| Chlorites | 0.1 |
| Hydroxides | 0.5 |
| Water | 7 |

Processes for preparing lithium hypochlorite compositions are found in U.S. Patents Nos. 2,590,794 (Robson), issued March 25, 1952; 2,534,781 (MacMahon), issued December 19, 1950; and 3,171,184 (Orazem et al.), issued March 2, 1965. The term "lithium hypochlorite" used in the specification and claims means "true" lithium hypochlorite, a compound of the formula LiOCl. Various lithium hypochlorite compositions contain varying amounts of LiOCl, as indicated herein.

### Calcium Hypochlorite

The calcium hypochlorite material for the purposes of the present invention is preferably a solid, dry calcium hypochlorite composition containing at least about 65 percent by weight of calcium hypochlorite and less than about 5 percent of water, the balance being inert materials usually resulting from the process of manufacture, e.g., sodium chloride, calcium hydroxide, chloride and carbonate. In a heretofore customary practice of calcium hypochlorite manufacture, the calcium hypochlorite is obtained as a slurry containing crystals of calcium hypo-chlorite dihydrate [$Ca(OCl)_2 \cdot 2H_2O$] in a mother liquor consisting essentially of an aqueous solution of calcium hypochlorite and sodium chloride. The slurry is filtered on a rotary vacuum filter to produce a cake that retains sufficient mother liquor to have a moisture content of 45 percent to 50 percent by weight. The wet cake, e.g., from an Eimco Filter, when dried directly yields the 70 percent calcium hypochlorite of commerce. However, if a higher concentration of calcium hypochlorite is desired, the wet cake may be washed with water to remove some of the mother liquor and then filtered or centrifuged or otherwise processed to separate further quantities of liquor and to form a wet solid which on drying produces granules which contain from about 85 to 90 percent by weight of calcium hypochlorite. The latter granules, as well as essentially 100% pure calcium hypochlorite, are also suitably used for formulating compositions of this invention.

The calcium hypochlorite content of the calcium hypochlorite granules used in formulating compositions of this invention is generally at least about 65 percent and preferably ranges from about 65 to about 80 percent by weight. The compound calcium hypochlorite contains about 100% available chlorine, thus a composition containing 65% calcium hypochlorite contains about 65% available chlorine.

For purposes of the present invention, commercial calcium hypochlorite preparations are generally suitable but specially high test compositions are sometimes desirably used. Commercial calcium hypochlorite as sold, usually contains at least about 65 percent available chlorine and, as manufactured, contains 71 to 73 percent of calcium hypochlorite and less than 5 percent water, preferably less than 1 percent water. A commercial calcium hypochlorite containing about 65 percent to about 72 percent available chlorine is marketed under the name "HTH" by Olin Mathieson Chemical Corporation. A typical analysis of HTH is as follows:

| Ingredient | Typical Weight % |
|---|---|
| Calcium hypochlorite Ca(OCl)$_2$ | 71 |
| Sodium chloride NaCl | 17 |
| Calcium hydroxide Ca(OH)$_2$ | 2.5 |
| Calcium carbonate CaCO$_3$ | 1.5 |
| Calcium chlorate Ca(ClO$_3$)$_2$ | 0.9 |
| Calcium chloride CaCl$_2$ | 0.8 |
| Water | 5.5 |

A process for preparing a calcium hypochlorite composition may be found in U.S. Patent 3,953,354 (Faust), issued April 27, 1976. Other suitable calcium hypochlorite compositions may be found in U.S. Patents No. 3,639,284 (Long and Sawhill), issued February 1, 1972, and 3,560,396 (Robson), issued February 2, 1971. The term "calcium hypochlorite" used in the specification and claims means "true" calcium hypochlorite, a compound of the formula $Ca(OCl)_2$. Various calcium hypochlorite compositions contain varying amounts of $Ca(OCl)_2$ as indicated herein.

### Diluent Salts

The solid compositions of this invention may additionally contain diluent salts. "Diluent salts" means salts other than lithium hypochlorite and calcium hypochlorite which may be compatibly incorporated into these solid compositions. This includes the diluent salts present in the commercial sources of lithium hypochlorite and calcium hypochlorite, as well as additional diluent salts which may be added in the preparation of the present compositions Any inorganic salt which is not reactive to hypochlorite bleach may serve as a compatible diluent salt in these compositions. Examples of suitable diluent salts include the alkali metal and alkaline earth chlorides, fluorides, sulfates, chlorates, orthophosphates, polyphosphates, pyrophosphates, nitrates, and mixtures thereof. Water-soluble salts, particularly the alkali metal salts, are especially preferred diluent salts.

Specific examples of diluent salts include sodium fluoride, sodium chloride, sodium sulfate, calcium chloride, sodium chlorate, calcium chlorate, sodium orthophosphate, sodium tripolyphosphate, sodium pyrophosphate, sodium nitrate, calcium nitrate, and mixtures thereof. The amount of diluent salts in the compositions of this invention can range from about 1% to about 90% by weight of the compositions, preferably from about 20% to about 80%, and most preferably from about 50% to about 80%.

The choice of diluent salts will affect the hydration or swelling of the compacted cakes on initial exposure to the aqueous environment. To minimize this swelling of the cake and to improve dissolution of the disinfecting composition, an especially preferred combination of diluent salts for the compositions of this invention is the mixture of sodium chloride and sodium sulfate. This mixture of diluent salts may comprise from about 99.5% to about 5% by weight of sodium chloride and from about 0.5% to about 95% by weight of sodium sulfate; preferably from about 95% to about 50% by weight of sodium chloride and from about 5% to about 50% by weight of sodium sulfate; and most preferably from about 70% to about 80% by weight of sodium chloride and from about 20% to about 30% by weight of sodium sulfate. This combination of diluent salts can be mixed with pure calcium and lithium hypochlorite, or with the commercial grades of calcium and lithium hypochlorite which already contain these and/or other diluent salts.

### Method of Preparation

The compacted solid compositions of this invention may be prepared by any conventional compacting procedure. For example, granules of lithium hypochlorite (e.g., Form 2) and granules of calcium hypochlorite (e.g., HTH) and any additional diluent salts are mixed together, and this mixture is then passed to a compacting machine. The granules are generally in a size range of from about 50 microns to about 1,000 microns prior to compacting.

The compacted solid can be formed by tabletting, "slugging," Chilsonating or otherwise converting the granular hypochlorite mixture into compacted forms.

The compacted solids can be formed, for example, in a conventional tabletting machine. The granular lithium hypochlorite and granular calcium hypochlorite formulation are initially weighed and then dry mixed together to produce a homogeneous mixture. Suitable diluent salts may be added in appropriate amounts at this point. This resulting mixture is then stamped into a compact cake. Compacting may be accomplished at pressures of from about 0.5 tons/square inch to about 200 tons/square inch, preferably from about 1.0 tons/square inch to about 50 tons/square inch, and most preferably from about 1.5 tons/square inch to about 5.0 tons/square inch. The compacting can be done on any conventional compacting apparatus, e.g., a Stokes Model R Tablet Press. The compacted cakes generally have a specific gravity of about 1.3 to about 2.3, preferably from about 1.5 to about 2.0.

The compacted blends of the invention are formed into shapes with dimensions appropriate to the desired use. For instance, cakes to be used in a dispenser for chlorine disinfectant in a toilet flush tank will be formed in the shape and size appropriate to fit the chamber or compartment of the dispenser which holds the cake.

## Method of Use

The compositions of this invention may be used in any aqueous medium such as swimming pools, wash basins, and the like, to provide a source of disinfecting chlorine. An especially preferred use is as a disinfectant chlorine source in toilet tanks. The compacted compositions are placed within a dispenser which is used within the flush tank of the toilet. Operable dispensers are described in U. S. Patent 3,952,339 (Baur et al.), issued April 27, 1976; U. S. Patent 3,778,849 (Foley), issued December 18, 1973; and U. S. Patent 3,769,640 (Castronovo), issued November 6, 1973. Various embodiments of a particularly preferred toilet tank dispenser are described in European Patent Application No. 78200257.0, Attorney's Docket No. 2502R, filed on October 20, 1978. These compacted cakes should be of a size to fill from about 10% to about 90% of the volume in the product compartments of these various dispensers, particularly from about 50% to about 80% of the volume of the product compartments of dispensers as described in the aforementioned Dirksing application, to accommodate hydration or swelling of these cakes on initial contact with water.

Water is introduced into the dispenser during a normal flush cycle of the toilet, contacting the composition in the dispenser. This water remains in the dispenser in contact with the composition between flushes, and during this time a portion of the composition dissolves in the portion of water thereby forming a relatively concentrated solution of hypochlorite. Preferably, all sides of the compacted compositions are exposed to the portion of water within the dispenser.

When the toilet is flushed, a portion of the concentrated hypochlorite solution in the dispenser is discharged into the toilet bowl along with substantially all the water in the flush tank.

Exemplary embodiments of the present invention are described as follows:

## EXAMPLE I

0005286

A solid, compacted cake of the following formula is prepared by mixing Form 2, HTH, NaCl and $Na_2SO_4$ in the indicated proportions and subjecting the granular mixture to a compaction pressure of about 2.5 tons per square inch on a Stokes Model R Tablet Press:

| Ingredient | Weight Percent (Approximate) |
|---|---|
| Form 2 | 27.2 (30% LiOCl) |
| HTH | 43.9 (65% Ca[OCl]$_2$) |
| NaCl | 21.7 |
| Na$_2$SO$_4$ | 7.2 |
| | 100.0 |

This composition has a $LiOCl:Ca(OCl)_2$ weight ratio of about 0.29:1, and an available chlorine level ($AvCl_2$) of about 38% to 39%. The cake has a specific gravity of about 1.7. The cake has dimensions of about 3.5 inches by about 2 inches by about 1/2 inch, for a total surface area of about 19.5 square inches and a volume of about 3.5 cubic inches. These particular dimensions of a cake are designed to fit into a toilet tank dispenser chamber having dimensions of from about 3.625 inches by about 2.25 inches by about 0.75 inches for a total volume of about 6.08 cubic inches. Such a dispenser is generally described in European application No. 78200257.0, (Attorney's Docket No. 2505R) incorporated by reference herein.

This compacted cake is designed to fit a product compartment of a dispenser generally similar to the embodiment described in Figure 12 of the aforedescribed Dirksing application. The mechanism of operation of this embodiment is described from Page 18, line 4, to Page 25, line 26, of that application. The cake thus fills about 60% of the volume of this compartment. All surfaces of the cake are exposed to the water which fills the remainder of the product chamber, and which resides in the chamber between flushes. A relatively concentrated solution of hypochlorite is therefore formed in this chamber.

On the flush, this concentrated solution of hypochlorite is discharged into the toilet bowl along with the flush water, providing a bowl concentration of the order of about 3 to about 25 parts per million (ppm) available chlorine ($AvCl_2$). The composition of this example exhibits excellent, steady chlorine release to the toilet over the duration of the life of the cake in the dispenser, which can be from about 2-8 weeks depending upon the frequency of the flushing of the toilet. The amount of chlorine released to the toilet is substantially unaffected by the temperatures of the water within the range of about 40°F to about 80°F.

Excellent results are also obtained when other inorganic diluent salts are substituted for sodium chloride and/or sodium sulfate in comparable amounts, including sodium fluoride, calcium chloride, sodium chlorate, calcium chlorate, sodium orthophosphate, sodium tripolyphosphate, sodium pyrophosphate, sodium nitrate, calcium nitrate, and mixtures thereof.

0005286

## EXAMPLE II

The hypochlorite formulation of Example I was compared against lithium hypochlorite formulations and calcium hypochlorite formulations for sustained release of active chlorine disinfectant in water. The three formulations tested and their available chlorine levels, were as follows:

### Composition A

100% by weight of Form 2, about 35% to 36% available chlorine ($AvCl_2$).

### Composition B

The composition of Example I, about 38% to 39% available chlorine ($AvCl_2$).

### Composition C

58.4% by weight of HTH, 31.2% by weight of sodium chloride, 10.4% by weight of sodium sulfate, about 38% to 39% available chlorine ($AvCl_2$).

Compositions A, B and C were all compacted into solid cakes of the same size, with the compacting procedure described in Example I.

Compositions A, B and C were each placed in the compartment of a toilet tank dispenser of the type described in Example I. The dispensers operate in the same manner as described in Example I. Parallel experiments were run wherein dispenser containing these three compositions were placed in the flush tanks of toilets which were fed with about 70°F water and toilets which were fed with about 40-45°F water. The toilets were flushed 200 times, simulating a home use cycle.

Between about 8 AM to about 9 AM all the toilets were flushed four times. From about 9 AM to about noon the toilets were not flushed. From about noon to about 1 PM the toilets were fushed four times. From about 1 PM to about 5 PM the toilets were not flushed. Between about 5 PM to about 6 PM the toilets were flushed four times. Between about 6 PM to about 11 PM the toilets were not flushed. Between about 11 PM to about midnight the toilets were flushed four times. Between about midnight to about 8 AM the toilets were not flushed. This simulated a home use cycle of a bathroom toilet bowl of about 16 flushes per day. This simulated home use cycle was carried on for about 12.5 days to simulate a total home use cycle of about 200 flushes.

The levels of available chlorine ($AvCl_2$) delivered to the toilet bowl were determined in parts per million (ppm) by the "Water Chex" method (manufactured by the Aseptic-Thermo Indicator Company, North Hollywood, California). Readings were taken after the second and third flushes of the 8 AM-9 AM flush sequence each day, and then averaged to determine the mean available chlorine level delivered by each composition at each water temperature. From this data, the ratio of available chlorine delivered in about 70°F water to available chlorine delivered in about 40-45°F water for each composition was then determined. The following table represents the average ratio of warm water available chlorine:cold water available chlorine for the three compositions during the 200 flush cycle:

| Formula | Ratio Warm Water $AvCl_2$ to Cold Water $AvCl_2$ |
|---|---|
| Composition A (LiOCl) | 0.76 |
| Composition B (LiOCl/Ca[OCl]$_2$ ratio about 0.29) | 0.98 |
| Composition C (Ca[OCl]$_2$) | 1.43 |

From the results of this experiment, it can be seen that Composition B, the composition of Example I, maintains a much more steady, sustained release of active chlorine disinfectant to the toilet water medium over a range of toilet water temperatures than either the LiOCl source alone or the $Ca(OCl)_2$ source alone. The present invention is able to accomplish this sustained chlorine delivery while at the same time employing a relatively small amount of lithium hypochlorite. This also assures consistent product life of the disinfectant source, regardless of water temperature.

0005286

WHAT IS CLAIMED IS :

1. A compact solid disinfecting composition containing from about 10% to about 107% by weight available chlorine, said available chlorine being supplied from a mixture of lithium hypochlorite and calcium hypochlorite wherein the weight ratio of lithium hypochlorite:calcium hypochlorite is from about 0.47:1 to about 0.17:1.

2. The composition of Claim 1 where the weight ratio of lithium hypochlorite:calcium hypochlorite is from about 0.37:1 to about 0.29:1.

3. The composition of Claim 1 which additionally contains from about 20% to about 80% by weight of inorganic diluent salt.

4. The composition of Claim 3 where the diluent salt is a mixture from about 99.5% to about 5% by weight of sodium chloride and from about 0.5% to about 95% by weight of sodium sulfate.

5. The composition of Claim 4 where the diluent salt comprises from about 70% to about 80% by weight of sodium chloride and from about 20% to about 30% by weight of sodium sulfate.

European Patent
Office

**EUROPEAN SEARCH REPORT**

0005286

Application number

EP 79 20 0171

## DOCUMENTS CONSIDERED TO BE RELEVANT

| Category | Citation of document with indication, where appropriate, of relevant passages | Relevant to claim |
|---|---|---|
| | GB - A - 1 175 749 (R. ALTIERI)  <br>  * Page 6, claims 1-7 *  <br><br>  -- | 1,2 |
| | FR - A - 1 582 688 (OLIN MATHIESON)  <br>  * Page 4, lines 15-31; page 5, lines 10-21 *  <br><br>  -- | 1,2 |
| A | GB - A - 1 126 108 (OLIN MATHIESON)  <br>  * Page 2, lines 118-127; page 7, claim 1 *  <br><br>  ---- | 1-5 |

**CLASSIFICATION OF THE APPLICATION (Int. Cl.²)**

A 61 L 13/00//
C 02 B 3/08
A 61 L 9/01

**TECHNICAL FIELDS SEARCHED (Int.Cl.²)**

A 61 L 13/00
          9/01
C 11 D 3/395
          17/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 21-06-1979 | PELTRE |

EPO Form 1503.1    06.78